# EUROPEAN PATENT APPLICATION

(11) **EP 3 488 896 A1**
(43) Date of publication of application: **29.05.2019**
(21) Application number: 17852468.2
(22) Date of filing: 18.09.2017
(51) Int. Cl.: A61N 1/04, A61N 1/32, A61N 1/40

(54) **DIATHERMY DEVICE**

(30) Priority: 26.09.2016 ES 201631251
(71) Applicant: Indiba, S.A., 08192 Sant Quirze del Valles (Barcelona) (ES)
(72) Inventor: CALBET BENACH, Jose, 08034 Barcelona (ES); RAMI MURILLO, Xavier, 08032 Barcelona (ES)
(74) Representative: Durán-Corretjer, S.L.P.
(86) International application number: PCT/ES2017/070616
(87) International publication number: WO 2018/055222

(57) **Abstract**

Diathermy device comprising a generator of alternating electric current and an electrode, the electrode and the generator being electrically connected, and characterised in that the electrode consists of a flexible glove comprising an inner layer of insulating material, an intermediate conducting layer connected to the generator and an outer layer made of an insulating material with a thickness of between 0.05 and 0.15 mm such that the outer layer has an impedance of less than 50 ohms for alternating electric currents with a frequency of between 100 kHz and 10 MHz, and in that the generator is configured to generate alternating electric currents at least at a frequency within said interval of 100 kHz and 10 MHz.

## Description

The present invention relates to a diathermy device. Diathermy is a treatment that uses electric currents to heat tissues. Diathermy is sometimes referred to as hyperthermia, although the term diathermy is preferable for distinguishing the temperature increase resulting from the passage of an electric current.

Documents ES287964U, US4944302A and ES8704742A1, from this applicant, disclosed the first electronic "recuperator" equipment fitted basically with various active metal electrodes with a polyamide insulating coating and a neutral passive electrode formed by a sheet of stainless steel or a handheld return electrode.

This technique has been very successful in physiotherapy, veterinary and human medicine, and cosmetic medicine, requiring the company to design several models with different ratings.

Subsequently, new, non-insulated stainless-steel electrodes have been incorporated into the various equipment, bringing clear benefits for these therapies, greater power absorbed by the body, faster and more beneficial treatments, and the possibility of alternating, combining and enhancing the two forms of treatment.

A known class of diathermy device comprises a generator, a neutral or return electrode and an active, application electrode, both being rigid and made of metal; the active electrode can be coated or not. The active electrode is smaller in size than the return one. To produce the diathermy effect, the generator is configured to produce alternating electricity at a fixed or selectable frequency of between 0.5 and 27 MHz.

One inherent limitation of diathermy treatments is the difficulty of applying them to anatomical areas that are irregular (joints, bone surfaces), small (lips, areas around the eyes) or difficult to access (facial areas like the jaw, chin, ears or nose) because it is physically impossible for the electrode to fit over the body surfaces to be treated, and ensure good contact.

PCT patent document WO2008/049947 discloses a hyperthermia device for electrotherapy, comprising a generator to which a neutral electrode is connected, for application to the patient's back, and a flexible, conducting active electrode, generally quadrangular in shape. The active electrode has straps with Velcro allowing it to be attached to the back of the hand or the forearm, depending on the size and shape of the electrode. The currents pass through the therapist's tissues, using the conductive properties of the human body, to the palm of his/her hand, and thence to the patient's body. To improve electrical contact, conducting gel is applied between the electrode and the therapist. In the case of the forearm, provision is made for the upper surface of the electrode, which is not in contact with the therapist, to have an insulating layer. In this case, the electrode is not adaptable to zones; rather, the therapist's hand is placed between the electrode and the patient. As well as solving the aforementioned problem, it has the advantage that it avoids superficial burns on the patient, since the therapist's hand also feels the heat. However, there is the problem that the currents pass through the therapist's body, and so it is not suitable for continuous use by a particular therapist.

The US patent document US5067478 discloses a glove for combined therapeutic pain-relief massage or acupressure and TENS therapy. TENS therapy uses the application of electric currents to stimulate the muscles electrically, activating them for the purposes of pain relief. The device has an electric current generator connected to a single electrode consisting of a glove made from a single piece of rubber, in which the inner layer is insulating and the outer layer (which will be in contact with the patient) is conducting. To perform TENS the pulses have to be produced at a frequency of up to 200 Hz, similar to the electrical pulses produced by the human nervous system. The inner layer is 0.762 mm thick, while the outer, conducting layer is 0.127 mm thick. The device is applied over the muscles, combined with massage. Another problem that this device has is that, to maintain the hygiene measures required in medical applications, a different glove has to be used for each patient, and the glove is expensive to manufacture. Another problem with this glove is that contact with the skin is unpleasant.

One object of the present invention is to disclose a device for applying diathermy therapies with an electrode that can be adjusted to anatomical areas of the body that are small, have an irregular surface or are difficult to access, such as the shoulders, knees, elbows, ankles, feet, ears, chin, jaw, nose and the area around the eyes.

Another object of the present invention is to disclose means permitting a novel, practical form of treatment, which applies both the capacitive and resistive form, i.e. the advantage of insulated electrodes and also non-insulated electrodes, i.e. stainless steel electrodes.

In particular, the present invention consists of a diathermy device comprising a generator of alternating electric current and an electrode, the electrode and the generator being electrically connected, and characterised in that the electrode consists of a flexible glove comprising an inner layer of insulating material, an intermediate conducting layer connected to the generator and an outer layer made of an insulating material with a thickness of between 0.05 and 0.15 mm, preferably between 0.05 and 0.1 mm, such that the outer layer has an impedance of less than 50 ohms for alternating electric currents with a frequency of between 100 kHz and 10 MHz, and in that the generator is configured to generate alternating electric currents at least at a frequency within said interval of 100 kHz and 10 MHz.

In the present invention, the term "glove" should be understood in a broad sense, i.e. as a covering that covers the hand with independent sheaths for each of the fingers, or where several fingers share one common sheath (as in a mitten).

The invention is based on the fact that, because the outer layer is very thin, electrical capacitance is established between the patient's skin and the conducting layer of the glove (between 1 nF and 10 nF). This means that, in the range of frequencies indicated, there is a sufficiently low impedance between the skin and the conducting layer to allow the passage of alternating current between the conducting layer and the skin.

There is electrical conduction through an insulated metal electrode because the current applied is alternating and high-frequency; if there was uniform direct current there would be no passage of circulating current.

As regards the intensity of alternating current, this depends on the frequency applied (in our case, from 400 kc/s to 1 MHz), as well as the voltage, the thickness and surface area of the insulating layer. In summary, the formula determining circulating current is proportional to 2.π.f.C.V, where "f" is frequency, "C" is capacitance and "V" is electrical voltage.

In addition, since the glove is flexible it can adapt to areas that are small, irregular and/or small and/or relatively inaccessible. Furthermore, it is especially advantageous that the currents are produced in a situation where there is capacitance between the skin and the intermediate layer, since this causes the effect of raising temperature to occur more selectively than in the situation where the conductive layer is in direct contact with the skin.

Nonetheless, the present invention is not limited to producing the effects of a capacitive electrode; rather, it also produces the effects of a resistive electrode. Indeed, the high capacitance represented by the fact that the conducting part of the glove is joined to the insulating part of the same glove, combined with the large area of contact that the glove has as an electrode, means that the circulating current is high enough to heat an area of the body, the foot or wrist joint, and by extension any other joint in the body, without the need to apply the glove directly to those zones, as happens with non-insulated stainless steel electrodes.

This demonstrates that the performance of the glove resembles the performance according to the resistive method, with respect to the form of operating, the capacitive method is understood *per* se since the metal part does not make direct contact with the body of the person receiving treatment. The advantages provided by the glove include the following:
1. There is no weight from the electrodes or the handle.
2. It perfectly fits the anatomy of very complex locations such as the shoulders, the knees, the fingers, the wrist, the face, etc.
3. Both the insulated (capacitive) electrode and stainless steel (resistive) electrode methods are introduced at the same time, which is more beneficial. In particular, the glove can be applied to the skin without movement, as with resistive electrodes.

The present invention provides for the glove to be single-component, or it can be formed in layers independent from each other.

For example, the glove could have its inner and intermediate layers joined together, forming a single part, while the outer one could be an independent, detachable layer. This allows it to be disposable. The outer layer could in this case take the form of a glove. In this way, the outer layer would be disposed of after each application, different outer layers being used for different patients, but a single assembly of inner layer and intermediate layer being used for each therapist.

In addition to the outer layer, the inner and intermediate layers could also be independent and capable of separation from each other. This also allows the inner layer to be replaced if it is damaged by sweat, or the same intermediate layer could be used by two different therapists, maintaining the necessary hygiene measures.

As already indicated, the device that is the subject-matter of the present invention, in any of its forms, as well as the glove-shaped electrode that forms part thereof, can be used for electrotherapy applications, particularly for diathermy applications and more particularly for treating areas of the human body that are small, irregular and/or difficult to access, both for simply cosmetic treatments and for therapeutic treatments.

For a better understanding, drawings of an embodiment of the subject-matter of the present invention are attached by way of explanatory but not restrictive example.

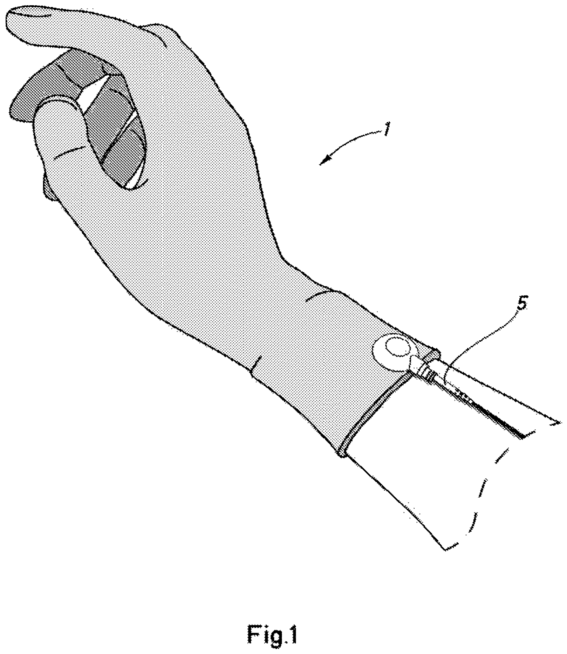

Fig. 1 is a perspective view of an exemplary embodiment of a glove belonging to a device according to the present invention.
Fig. 2 is a longitudinal section in the area of one of the fingers of the glove in Fig. 1, applied to the skin of a patient or person receiving treatment.

The figures show an embodiment of a glove 1 belonging to the device according to the present invention applied to the hand of a therapist. The glove is connected, via a suitable electrical cable 5, to a diathermy current generator, not shown in the figures. In particular, the diathermy current generator of the present invention will be configured so as to generate alternating electric current at a frequency of between 0.1 MHz and 10 MHz. The generator could be of a known type, and is therefore not shown in the figures.

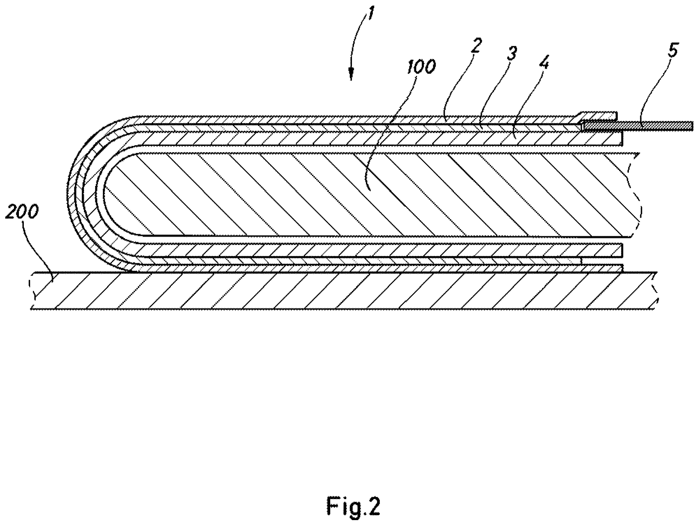

Fig. 2 shows a longitudinal section of the glove that is the subject-matter of the present invention, in the region of the therapist's finger 100. The drawing is schematic as regards the thickness of the layers and the location of the connection with the cable 5. As can be seen, the glove is composed of three layers, an inner, insulating layer 4, the purpose of which is to insulate the therapist from the currents, an intermediate, conducting layer 3 connected to the generator via the cable 5 and an outer layer 2 made of an insulating, but very thin, material. In particular, the thickness of the outer layer 2 will be between 0.05 and 0.15 mm, preferably less than 0.10mm, for example 0.07mm. Electrical capacitance is thus produced between the skin of the patient 200 and the intermediate layer 3. At the operating frequencies of the present invention, this means that the impedance of the outer layer is low enough (less than 50 ohms) for the thermal effect to be produced.

The insulating inner layer 4 is thicker, for example, between 0.5 mm and 1 mm, or more.

The glove can have the three layers 2, 3, 4 integrated, i.e. joined together, forming a single glove.

It is also possible for the inner layer 4 and intermediate layer 3 to be joined together, forming a single glove and for the outer layer 2 to take the form of an independent glove that can be disposed of after treatment.

It is also possible for each of the layers 2, 3, 4 to take the form of an independent glove layer. In this case, the therapist would first have to put on the glove corresponding to the inner layer 4, then the glove corresponding to the intermediate layer 3 and finally the glove corresponding to the outer layer 2.

The glove of the present invention has clear and unquestionable advantages for direct application by hand.
- The first is that the glove uses both the capacitive and resistive methods owing to the thickness of the outer insulator of the glove, and a greater and more noteworthy benefit is achieved, also with greater speed over time.
- Another advantage of the glove is that the heating of the hand is not bothersome, as happens with direct application; rather, the degree of heating on the hand can be selected.
- Finally, no handle or electrode needs to be supported. With the glove, the degree of heating of the hand can be regulated from feeling the same degree of temperature as the patient to not feeling anything.

Although the invention has been presented and described with reference to embodiments thereof, it will be understood that these are not restrictive of the invention, and therefore multiple structural or other details may vary, as may be obvious to experts in the sector after interpreting the matter disclosed in the present description, claims and drawings. Therefore, all variants and equivalents will be included within the scope of the present invention if they can be considered to be comprised within the most extensive scope of the following claims.

## Claims

1. Diathermy device comprising a generator of alternating electric current and an electrode, the electrode and the generator being electrically connected, and **characterised in that** the electrode consists of a flexible glove comprising an inner layer of insulating material, an intermediate conducting layer connected to the generator and an outer layer made of an insulating material with a thickness of between 0.05 and 0.15 mm such that the outer layer has an impedance of less than 50 ohms for alternating electric currents with a frequency of between 100 kHz and 10 MHz, and **in that** the generator is configured to generate alternating electric currents at least at a frequency within said interval of 100 kHz and 10 MHz.

2. Device according to claim 1, **characterised in that** the outer layer has a thickness of between 0.05 and 0.1 mm.

3. Device according to claim 1 or 2, **characterised in that** the outer layer is a detachable independent layer.

4. Device according to claim 3, **characterised in that** the inner and intermediate layers are independent and can be separated from each other.

5. Device according to any of claims 1 to 2, **characterised in that** the aforementioned layers are joined permanently, forming a single part.
